# EUROPEAN PATENT APPLICATION

(11) **EP 2 420 277 A1**
(43) Date of publication of application: **22.02.2012**
(21) Application number: 10763994.0
(22) Date of filing: 15.04.2010
(51) Int. Cl.: A61M 5/178, A61M 5/31

(54) **DEVICE FOR COVERING A SYRINGE AND NEEDLE IN ORDER TO ALLEVIATE THE FEAR AND ANXIETY EXPERIENCED DURING PEDIATRIC MEDICAL AND ODONTOLOGICAL PROCEDURES, SUCH AS THE ADMINISTRATION OF ANESTHETICS AND THE LIKE**

(30) Priority: 15.04.2009 BR PI0901236
(71) Applicant: Sociedade Educacional Uberabense, CEP: 38.010-200, Uberaba - MG (BR)
(72) Inventor: HUEB DE MENEZES OLIVEIRA, Maria Angélica, CEP: 38.050-030 Uberaba - MG (BR); HUEB DE MENEZES, Fernando Carlos, CEP: 38.020-060 Uberaba - MG (BR); BONFIM OLIVEIRA, Carlos Roberto, CEP: 38.082-043 Uberaba - MG (BR)
(74) Representative: Schwahn, Hartmut
(86) International application number: PCT/BR2010/000125
(87) International publication number: WO 2010/118491

(57) **Abstract**

Device for covering a syringe and needle in order to alleviate the fear and anxiety experienced during pediatric medical and odontological procedures, such as the administration of anesthetics and the like. A device for use, in particular, by a pediatric odontologist for administering anesthetics, or by other health care professionals for administering an injectable medicament, vaccines, collecting blood, etc., **characterized by** comprising a module for covering the syringe and needle (1, 9a, 9b, 9c, 9d, 9e and 9f), and by a needle guide that prevents the needle from breaking when an anesthetic (2) is injected, facilitating a positive behavior in the pediatric patient being treated in the medical or odontological clinic, and eliminating the fear, anxiety and discomfort of such a patient during restoration and surgical procedures.

## Description

This patent application relates to a device for covering syringes and needles for overcoming fear and anxiety in pediatric medical and dental procedures during the administration of anesthesia and other medicines, to be used especially by the pediatric dentist during the execution of anesthesia procedures and by other health professionals for injectable drug delivery, vaccinations, blood collection, etc.

Pain control through the overcoming of fear is one of the most important aspects to encourage the pediatric patient to behave positively in the dental clinic (Wilson,1996; WALTER; FERELLI, ISSAO, 1999; ROULET, GUEDES-PINTO, 2000).

However, the acceptance of treatment for this type of patient is still a challenge for the dentist working with children, because the administration of local anesthesia causes many children to avoid dental consultation and treatment (JOHNSON; PRIMOSCH, 2003).

Because of this, aspects that relate to painful phenomena have received special attention from professionals who specialize in child development in the field of healthcare.

The method in dentistry most commonly used to avoid pain is the inhibition of the channels that transmit nerve impulses through the administration of local anesthetics near the nerve endings involved. The local anesthetic prevents the depolarization of nerve fibers in the area of absorption, preventing them from transmitting any nervous impulse beyond that point (Bennett, 1989).

Local anesthesia is effective for dental procedures, but the appearance of the anesthetic needle and syringe causes fear and anxiety in many patients, particularly in pediatric dentistry.

Problems with patient management of children are often associated with age, i.e. younger children of preschool age are more challenging, and are compounded by the need for invasive medical procedures such as anesthesia, restorations, pulpotomies, pulpectomies, surgery, etc.

In dentistry, these procedures often lead to the manifestation of fear and of pain. Thus, the use of anesthetics, applied with syringes and needles, is essential in most cases.

Patients are afraid to go to the dental clinic because of the fear of the application of anesthesia, specifically the syringe and needles which cause pain, especially in the area of pediatric dentistry.

Pain control during pediatric dentistry is fundamentally important not only to a successful treatment but also to the relationship between the professional and the patient.

Local anesthetics aid treatment because they decrease the anxiety and discomfort experienced by the young patient during restorative and surgical procedures.

Therefore, certain aspects should be observed in pediatric dentistry, such the psychological preparation of the patient, topical anesthesia, pharmacological considerations of local anesthetics, anesthetic techniques, local anesthetic toxicity, complications of local anesthesia and new technologies related to local anesthesia.

The psychological preparation of the pediatric patient is fundamental, because the child should cooperate with the dentist during the application of the anesthetic in order to avoid any type of accident. (YAP AUJ, ONG, G., 1996; ROULET PLBC , et al 1997; MCDONALD et al, 1998; AZEVEDO AM et al, 2001).

The local anesthetic maybe applied painlessly, depending on the experience of the dentist and the collaboration of the patient.

Simply seeing a needle can initiate sensations of fear and anxiety in the child, in some cases even resulting in a panic attack, making dental treatment impossible. Therefore displaying the syringe and needle is uncalled for in most cases.

The physiology of fear is set off from the moment that a child enters into visual contact with objects that frighten the child. Upon seeing such objects, the eyes send a stimulus to the brain, in the form of electrical signals to the relevant structures that initiate a panic reaction to the fear causing stimulus, which are the cerebral amygdalae, located in the temporal lobes region.

The amygdalae send a signal to the hypothalamus, the area that controls the metabolism, in order to intensify the production of adrenalin, noradrenalin and acetylcholine.

Thus, in seconds, the release of these substances triggers several changes in the individual's body, through the activation of the sympathetic autonomic nervous system.

The type and intensity of physiological response and emotions vary, but the organism can be fully mobilized and experience some of the following systemic reactions due to panic:
a) Change in secondary blood flow, produced for the heart, muscles and central nervous system so that the individual may have clear thinking and quick action;
b) Liver: the release of sugar reserves by the liver (gluconeogenesis) to supply the muscles;
c) Eyes: the pupils dilate for better vision;
d) Heart: increased heart rate and, consequently, shortness of breath and panting to provide more oxygen;
e) Stomach may ache due to the increased production of acetylcholine. There is increased release of gastric juices, speeding up digestion and the transformation of food into energy (CORREA MSNP, 2002).

In pediatric dentistry, local anesthesia should always be performed, even in the simplest procedures, such as the placement of a brace during the performance of an isolate.

In practice, the methods currently used to try to minimize the difficulties of treating children include only the use of a cartridge syringe or placing a roll of cotton around the tip of the needle in an attempt to distract the patient.

The recommended in dentistry practice is that the dentist should try to move the syringe behind the child's head and below their line of vision, when applying anesthesia to the oral cavity of the patient.

The other method of trying to hide a needle is to put a roll of cotton around it. However, both of these maneuvers are inadequate, because the child can see the syringe and the needle, whose shape will frighten them. In addition there is a risk that the cotton may fall during the procedure, frustrating the attempt to deceive the patient.

The image of the needle for the child patient is especially traumatic, interfering with treatment in subsequent visits.

Another factor to be considered in relation to the image of the needle is that fear can lead a child to move their head suddenly, causing the needle already inserted in the oral mucosa to break, a complication that could be avoided if the child does not make eye contact with the needle.

Besides fear, children can also suffer from the feeling of anxiety. The definition of anxiety refers to unease without cause or in relation to any context of danger, and it has to do, in fact, with a unconscious psychological cause (DE JONG, A 1988), in that most children's fears come from the imagination.

The device for covering the syringe and needle to overcome fear and anxiety in pediatric medical and dental procedures for the administration of anesthesia and other medicines of the present application has been developed to dispel fear and anxiety in children, especially of the anesthetic syringe and needle used during dental treatment.

Currently, in the market and in practice, there is nothing that administers a local anesthetic to the child effectively, safely and painlessly, ensuring the positive response of the pediatric patient in the dental office, avoiding fear, anxiety and discomfort to the patient during restorative and surgical procedures.

To be successful in child patient management it is necessary to connect with the child's world of imagination in order to establish a trusting patient / professional relationship.

When a dental professional enters into the child's world by showing them objects that are of interest, the patient is cooperative.

The experience of clinical pediatric dentistry at the University of Uberaba has revealed that even before receiving its first dental consultation, a child comes with a preconceived negative opinion regarding dental treatment, due to remarks from their parents or other adults, a fact that justified the research and development of the present device.

For research purposes the device for covering syringes and needles for overcoming fear and anxiety in pediatric medical and dental procedures of the present invention was tested for two months at the pediatric dentistry clinic of the University of Uberaba, with approximately three hundred (300) children, producing evidence that the children's acceptance of treatment improves when using the device, optimizing their care and restoration of oral health.

When anesthetized using the device to overcome fear, the child relates to his imaginary world because of its function and form, facilitating a peaceful treatment free from anxiety and without fear, because the objects of fear are out of the visual field.

Children who were anesthetized with the device for covering syringes and needles for overcoming fear and anxiety in pediatric medical and dental procedures of the present invention at the clinic of the University of Uberaba no longer agree to be anesthetized with a standard cartridge syringe and ask the professional to use the Device, because, according to the patients, "it is better, because the tooth goes to sleep without pain."

It can be seen that pain can be activated by suggestion and when the child is fearful of an anesthetic syringe, they can withdraw, tense their muscles, move their head and make treatment difficult or impossible.

The device for covering syringes and needles for overcoming fear and anxiety in pediatric medical and dental procedures of the present invention is a great aid for the professional working with children, disguising the shape of syringes used for injection.

The unique and inventive design of the Device is made by placing both the syringe and the needle together into a sleeve to help overcome fear and anxiety, transforming its appearance from the conventional system of injection, which is easily recognized by patients, to a creative and novel instrument.

The device for covering syringes and needles for overcoming fear and anxiety in pediatric medical and dental procedures of the present invention is characterized by a sleeve module for covering the syringe and needle (1) and by a needle guide that prevents it from breaking during anesthesia delivery (2).

When using the Device, the dentist or other health care professional inserts the syringe (3) inside the lining of the syringe sleeve module (4a and 4b), removes the needle cap and then places the device inside the patient's oral cavity for the administration of anesthesia, using pressure on the plunger (5) and the handles of the supporting module which retracts the sleeve, exposing the needle (6).

The device can be fitted with hypodermic syringes or cartridges, and at the front of the device there are two supporting handles (6), a support housing for the syringe (7) and a bellowed or bulb section (8a or 8b) which enables the retraction of the device, the projection of the needle and puncturing at the insertion point of anesthesia or other medication.

A needle guide is located on the inside of the rear of the device (2) in order to prevent the needle from breaking during anesthetic injection, which can either be attached or prefabricated with the module. The top of the device can also have a transparent version to allow the dentist or other professional to verify the amount of anesthesia or other medication being injected.

At the end of the injection, the dentist or other professional releases pressure on the plunger and on the module's support handles and the needle automatically retracts inside the device due to the expansion of the bellows or bulb of the handle.

This device may be produced in polymer or other flexible materials and in bright colors, promoting ecological associations by the use of animal forms from nature such as the alligator, whale, eagle, hummingbird and centipede, etc. (9a, 9b, 9c, 9d, 9e) or in the case of infants, a feeding bottle (9f).

Therefore, the health professional can work more safely, free from concerns related to potential trauma caused to patients resulting from seeing the syringe and needle or by the fear of anesthesia.

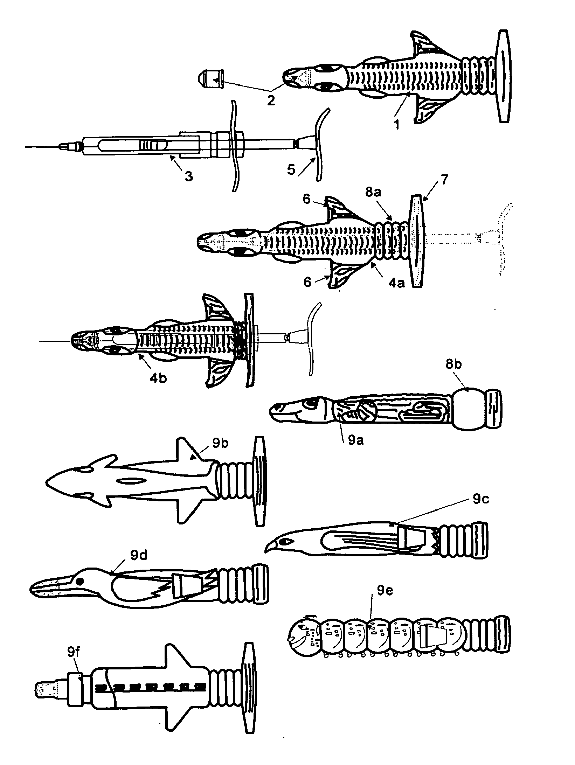

## Claims

1. Device for covering syringes and needles for overcoming fear and anxiety in pediatric medical and dental procedures during the administration of anesthesia and other medicines **characterized by** comprising a module for covering the syringe and needle (1, 9a, 9b, 9c, 9d , 9e and 9f) and a guide for the needle that prevents its breaking during injection for anesthesia and medicament delivery (2).

2. Device according to claim 1 **characterized by** the fact that it comprises a sleeve module for covering the syringe and needle (1), whose dimensions should allow accurate fitting with hypodermic syringes or cartridges, which
has two handle supports (6), a housing support for the syringe (7) and a bellow or bulb section used to retract the sleeve in order to project the needle (8a or 8b) and puncture the insertion point of the anesthesia or other medicines.

3. Device according to claim 1 **characterized by** the fact that it comprises a needle guide that prevents the needle from breaking during injection (2), whose dimensions are always proportional to the measurements of the covering module, allowing for the correct fitting of hypodermic syringes or cartridges.

4. Device according to anyone of claims 1 to 3, **characterized by** the fact that it is for use in a process of overcoming fear and anxiety in pediatric medical and dental procedures during the administration of anesthesia or other medicines by covering the syringe and needle, allowing for peaceful treatment without anxiety and without fear, because the objects of fear remain outside the patient's visual field.
